(19) Europäisches Patentamt
European Patent Office
Office européen des brevets

(11) Numéro de publication: **0 043 811**
**B1**

(12) # FASCICULE DE BREVET EUROPEEN

(45) Date de publication du fascicule du brevet: **20.03.85**

(21) Numéro de dépôt: **81870032.0**

(22) Date de dépôt: **01.07.81**

(51) Int. Cl.⁴: **C 07 D 471/16,** A 61 K 31/435 // (C07D471/16, 221/00, 221/00, 209/00)

(54) **Nouvelles indolonaphtyridines et leur utilisation en tant que médicaments.**

(30) Priorité: **03.07.80 BE 884145**

(43) Date de publication de la demande: **13.01.82 Bulletin 82/2**

(45) Mention de la délivrance du brevet: **20.03.85 Bulletin 85/12**

(84) Etats contractants désignés: **AT BE CH DE FR GB IT LI LU NL SE**

(56) Documents cités: **FR - A - 2 376 150**

(73) Titulaire: **OMNICHEM Société anonyme, 12 Avenue de Broqueville, B-1150 Bruxelles (BE)**

(72) Inventeur: **Hannart, Jean Alfred Alphonse, avenue d'El Pirere 25, B-1302 Dion Valmont (BE)**

(74) Mandataire: **Van Malderen, Michel et al, p.a. Freylinger & Associés 22 avenue J.S. Bach (bte 43), B-1080 Bruxelles (BE)**

**EP 0 043 811 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un composé polycyclique comportant un groupement indolique et s'étend à l'application thérapeutique de ce composé à titre de principe actif de médicaments, en particulier pour le traitement des insuffisances cérébrovasculaires et des états dépressifs.

La présente invention porte plus particulièrement sur l'un des dérivés de l'hexahydro-1,2,3,3a,4,5-6H-indolo (3,2,1-de) (1,5) naphtyridine de formule

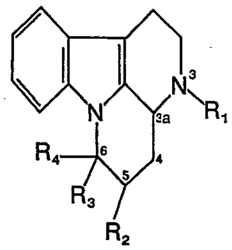
(I)

sous forme de base ou de sel d'addition d'acide, dans laquelle $R_1$ représente un atome d'hydrogène ou un groupe alkyle comportant de 1 à 6 atomes de carbone $R_4$ représente un atome d'hydrogène, et soit $R_2$ représente ensemble avec $R_3$ une liaison carbone–carbone supplémentaire; soit $R_3$ représente un groupe hydroxyle et $R_2$ représente un atome d'hydrogène. Le composé de l'invention est le dérivé de formule (I) dans laquelle $R_1$ = éthyle, $R_4$ = hydrogène et $R_2$ et $R_3$ représentent ensemble une liaison carbone–carbone supplémentaire.

Les composés I peuvent se présenter sous forme d'isomères optiques ou de mélange racémique.

En effet ces composés comportent un atome de carbone 3a asymétrique. Toutes ces formes isomères relèvent de la présente invention.

Le squelette carboné de ces dérivés correspond à celui de la vincamine (formule II) dans lequel le cycle D et les groupes carbométhoxy 16 et éthyle en position 20 sont absents.

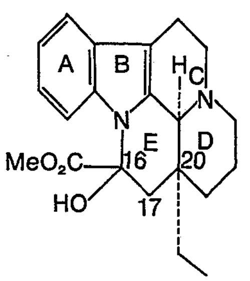
(II)

Le brevet belge 870 887 (US 4 200 638) décrit des composés de formule I dans laquelle $R_4$ est un groupe carbométhoxy et $R_2$ et $R_3$ forment ensemble une liaison carbone–carbone supplémentaire. On les appelle également chano-déséthylapovincamines.

Le brevet belge 857 816 (US 4 218 453) décrit et revendique des composés de formule I dans laquelle $R_2$ représente un atome d'hydrogène et $R_3$ et $R_4$ représentent ensemble un atome d'oxygène (hexahydrocanthinones-6).

Le FR-A-2 376 150 décrit des composés de formule I dans laquelle $R_1$ représente un groupe alkyle et chacun de $R_2$, $R_3$ et $R_4$ représente un atome d'hydrogène.

Les composés de formule I sont obtenus avantageusement par condensation d'une tryptamine et d'un aldéhyde fonctionnalisé, suivi par une modification chimique classique (déshydratation, réduction, alkylation).

Les composés I possèdent des propriétés thérapeutiques intéressantes et constituent par ailleurs des intermédiaires pour la synthèse de dérivés possédant également de telles propriétés.

Pour préparer un composé de formule (I) dans laquelle $R_4$ = H; $R_3$ = H, OH ou ensemble avec $R_2$ une liaison C–C supplémentaire, une $N_b$-alkyl tryptamine de formule III où $R_1$ a la signification précédemment précisée, est condensée avec un composé de formule

$$R{-}O{-}CO{-}CH_2{-}CH_2{-}CO{-}R'$$

où R désigne un groupe alkyle en $C_1$–$C_6$ notamment méthyle ou éthyle et R′ peut notamment désigner un atome d'halogène pour obtenir un ester d'acide N-2-(indolyl-3-éthyl)-succinamique de formule

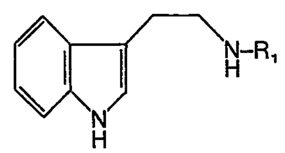
(III)

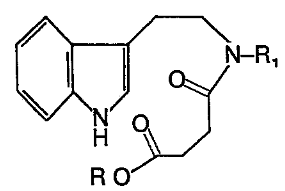
(IV)

où $R_1$ et

R ont les significations données précédemment.

Ensuite, l'ester de formule IV est traité par un réactif connu pour provoquer la réaction de cyclisation dite de BISCHLER NAPIERALSKI, tel que l'oxychlorure de phosphore, pour donner un dérivé tricyclique de formule

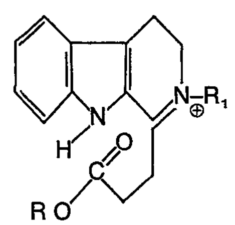
(V)

dans laquelle R et $R_1$ ont les significations données précédemment, puis ce dérivé est réduit dans le méthanol au moyen d'un borohydrure alcalin pour fournir une 1,2,3,4-tétrahydro-1-(2-carboalkoxy éthyl)-β-carboline éventuellement N-substituée de formule

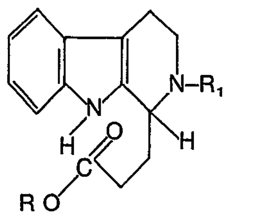

(VI)

dans laquelle $R_1$ et R ont la signification donnée plus haut.

Le composé ainsi obtenu est alors cyclisé en hexahydrocanthinones de formule générale VII.

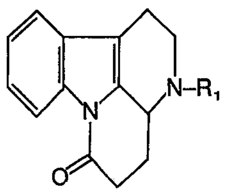

(VII)

Alternativement, pour obtenir une canthinone de formule VII, une N-b alkyltryptamine de formule III où $R_1$ a la signification précédemment précisée, est condensée avec l'acide β-formylpropionique dans un hydrocarbure aromatique tel que le benzène, le toluène. Après un reflux de 4 à 5 heures, le solvant est évaporé et le résidu dissous dans l'acide acétique est chauffé à reflux pendant 2 heures, puis la solution est diluée à l'eau et de nouveau chauffée à reflux pendant 2 heures. Lorsque la réaction est terminée, le milieu est refroidi, dilué à l'eau, alcalinisé par de la soude ou de la potasse caustique et extrait par un solvant organique. Par évaporation et cristallisation, on obtient les composés de formule générale VII.

Cette réaction peut être représentée par le schéma suivant:

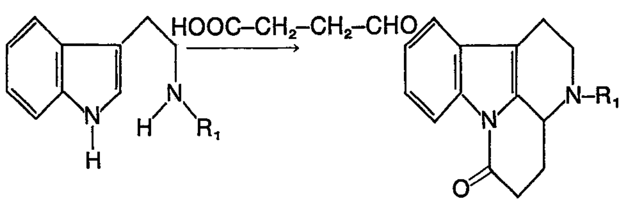


(III) (VII)

Les canthinones VII sont ensuite réduites en canthinols (Id, $R_3$ = OH, $R_4 = R_2$ = H) puis déshydratées pour fournir les canthènes (Ib, $R_4$ = H, $R_2$ et $R_3$ = liaison supplémentaire).

La réduction en canthinol est effectuée par un hydrure tel que $LiAlH_4$ dans un solvant inerte, par exemple le tétrahydrofuranne ou le diglyme. Ces alcools peuvent être éventuellement isolés sous forme optiquement active. De plus, le mélange réactionnel contient les deux diastéréoisomères possibles du canthinol (αOH et βOH).

Ceux-ci peuvent être isolés par cristallisation fractionnée.

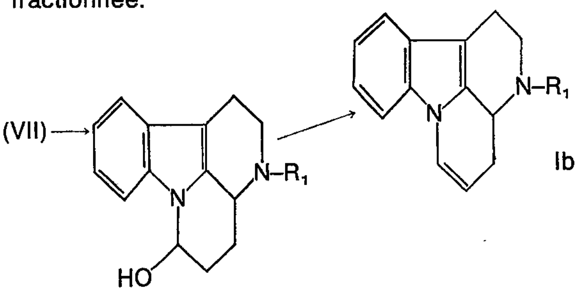

(VII)

Ib

Ia

Les hydroxy-6-hexahydro-(1,2,3,3a, 4,5)-indolo (3,2,1,-de) (1,5)naphtyridines fournissent les produits de déshydratation correspondants en présence d'un agent chlorant tel que le $POCl_3$ dans un solvant organique inerte tel le benzène ou le chlorure de méthylène et en présence d'une base telle la triéthylamine ou la pyridine.

Ces composés fournissent par hydrogénation en présence d'un catalyseur à base de métaux, par exemple le palladium, le platine ou le nickel, les composés correspondants saturés en position 5 et 6 (I, $R_2 = R_3 = R_4$ = H). On utilisera de préférence le palladium sur un support carboné, tel le charbon.

Exemple 1

Hydroxy-6-méthyl-3-hexahydro-1,2,3,3a,4,5-3H-indolo (3,2,1-de) (1,5)naphtyridine

(Ia, $R_1$ = méthyl)

2,1 g d'hydrure de lithium aluminium sont mis en suspension dans 200 ml de THF anhydre. Le mélange est refroidi dans un bain de glace et 6 g de 3-méthyl-1,2,3,3a,4,5-hexahydrocanthinone-6 en solution dans 150 ml de THF anhydre sont ajoutés goutte à goutte.

Après addition, le milieu est agité pendant 30 minutes à la température ambiante et l'excès d'hydrure de lithium aluminium est détruit par addition de THF saturée d'eau. Après filtration, le filtrat est évaporé à sec et le résidu est repris par le chlorure de méthylène. La solution organique est lavée à l'eau, séchée sur sulfate de magnésium et concentrée jusqu'à cristallisation.

On obtient ainsi 4,2 g de cristaux (mélange de 4 isomères) (OH 6α, H 3aα, OH 6α, H 3aβ; OH 6β, H 3aα; OH 6β, H 3aβ).

Caractéristiques physiques:

Point de fusion: 150 °C.

Spectre I.R. (KBr, $cm^{-1}$):

absence de bande à 1700.

Spectre U.V. (c = 2,11 $10^{-5}$ mol/1, MeOH, nm, log ε):

217 (4,34); 227 (4,52); 246 (2,65); 275–282 (3,83); 290 (3,69).
Spectre de masse:
$M^+$ 242 pour $C_{15}H_{18}ON_2$
Analyse élémentaire:
Calculé
% C 74,35 % H 7,48 % N 11,56
Observé
% C 74,12 %H 7,37 % N 11,61

Exemple 2
Hydroxy-6-éthyl-3-1,2,3,3a,4,5-hexahydro-6H-indolo (3,2,1-de) (1,5)naphtyridine
(Iα, $R_1$ = éthyl)

12,4 g de 3-éthyl-1,2,3,3a,4,5-hexahydrocanthinone-6 traités comme décrit dans l'exemple 1 fournissent 10,5 g de cristaux (mélange de 4 isomères).

Caractéristiques physiques:

Point de fusion: 165 °C.
Spectre I.R. (KBr, $cm^{-1}$):
absence de bande vers 1700.
Spectre U.V. (c = 1,98.$10^{-5}$ mol/1, MeOH, nm, log ε):
218 (4,39); 227 (4,53); 246 (3,01); 279–281 (3,83); 290 (3,68).
Spectre de masse:
$M^+$ = 256 calculé pour $C_{16}H_{20}ON_2$.
Analyse élémentaire:
Calculé
% C 74,96 % H 7,86 % N 10,92
Observé
% C 74,87 % H 7,91 % N 10,89

Exemple 3
Ethyl-3-tétrahydro-1,2,3,3a-4H-indolo (3,2,1-de) (1,5)naphtyridine (Ib, $R_1$ = Et)

18,3 g de hydroxy-6-éthyl-3-tétrahydro-1,2,3,3a-6H-indolo (3,2,1-de) (1,5)naphtyridine (0,0714 mole) sont mis en suspension dans 300 ml de benzène anhydre et 300 ml de triéthylamine. Le mélange est refroidi à 0 °C à l'aide d'un bain de glace et 18 ml de $POCl_3$ sont ajoutés goutte à goutte. Après une heure à 0 °C, le mélange réactionnel est versé dans 5 litres d'eau. La phase benzénique est décantée et la phase aqueuse est extraite avec deux fois 300 ml de benzène.

Les phases benzéniques réunies sont lavées à l'eau, séchées sur sulfate de magnésium et, après filtration, évaporées jusqu'à siccité. On obtient ainsi 15,3 g d'huile jaune qui cristallise.

Les cristaux sont dissous dans un minimum de THF anhydre et le chlorhydrate est précipité par addition d'éther saturé en HCl. Par recristallisation dans le méthanol, on obtient 11 g (0,034 mole) de chlorhydrate et éthyl-3-tétrahydro-1,2,3,3a-4H-indolo (3,2,1-de) (1,5)naphtyridine (rendement: 45%).

Caractéristiques physiques:

Point de fusion: 210–211 °C (chlorhydrate).
Spectre I.R. (film, NaCl, base, $cm^{-1}$):
2980, 1650, 1470, 1450, 1310, 730.
Spectre U.V. du chlorhydrate
(c = 3,74.$10^{-5}$ mol/1, MeOH,
221 (4,31); 223 (4,32); 235 (4,01); 256 nm, log ε): (4,40); 284 (3,63); 300 (3,82); 304 (3,79); 309 (3,84).
Spectre de masse (m/e):
$M^+$ = 238 calculé pour $C_{16}H_{18}N_2$
Spectre RMN de la base ($CDCl_3$ δ en ppm):
7,6–6,8 (massif, 5H); 5,2 (t dédoublé 1H); 1,2 (t, 3H).

L'invention comprend également les applications industrielles et notamment pharmaceutiques du produit Ib décrit ci-dessus. Les composés de formule (I) ont été soumis à des essais pharmacologiques qui ont révélé des propriétés intéressantes notamment des propriétés antianoxiques, psychotropes et oxygénatrices cérébrales.

Toxicité aiguë

Les composés Ia, Ib et Ic ont été administrés sous forme de chlorhydrate par voie intraveineuse à des souris de souche Charles River. Les doses létales 50% ($DL_{50}$) ont été déterminées graphiquement selon la méthode de Lichtfield et Wilcoxon (J. Pharmacol. Exp. Thérap. 1946, 96, 99).

Epreuve d'anoxie hypobare chez la souris

Des souris de même sexe, de souche Charles River, pesant environ 20 ± 2 g sont réparties en trois lots de 10 animaux. Les lots 1 et 2 comportent les animaux traités, c'est-à-dire ayant reçu la substance à tester de l'invention. Un troisième lot comporte des animaux témoins.

Les composés étudiés sont administrés p.o. 30 minutes avant l'essai. Les animaux sont placés dans une atmosphère appauvrie en oxygène par réalisation d'un vide partiel (190 mm Hg, correspondant à 5,25% d'oxygène), ces conditions étant obtenues en 30 secondes. On mesure le temps de survie des souris au moyen d'un chronomètre. Les agents capables de favoriser l'oxygénation tissulaire et en particulier cérébrale, entraînent un accroissement du temps de survie et l'on peut exprimer par la dose efficace 50% ($DE_{50}$), la dose qui augmente de 50% le temps de survie moyen des animaux placés dans les conditions décrites ci-dessus.

L'indice vincaminique correspond au rapport de la $DL_{50}/DE_{50}$ du produit à la $DL_{50}/DE_{50}$ de la vincamine.

Le tableau suivant rassemble les doses létales et les indices vincaminiques observés pour divers composés décrits dans la présente invention.

On peut en conclure la supériorité de ces nouveaux dérivés comparativement à la vincamine.

| $R_1$ | $R_4$ | $R_3$ | $R_2$ | $DL_{50}$ iv mg/kg | ind. vincam. hypobare |
|---|---|---|---|---|---|
| Et | H | OH | H | 21 | 2 |
| Et | H | liaison | | 46 | 2 (Ib) |
| Et | H | H | H | 14 | 2 |

Chez le chien anesthésié au Nembutal, les composés de l'invention, en particulier Ib, augmentent

les débits sanguins artériels des artères fémorales et vertébrales et stimulent de façon marquée (± 50%) la consommation apparente du cerveau en oxygène. L'effet sur la fréquence cardiaque est par ailleurs peu marqué, contrairement au cas de la vincamine.

Le canthène Ib s'est en outre révélé très actif au test à la ptose palpébrale induite par la réserpine chez la souris. Il ne présente cependant aucune caractéristique d'inhibiteur de monoamine oxydase (IMAO), caractéristique peu souhaitable pour une application en tant que antidépresseur en gérontologie. La combinaison des propriétés antianoxiques et antidépressives confèrent aux composés de l'invention un intérêt thérapeutique original.

En vue de leur application en thérapeutique, le composé de l'invention se présentera sous forme de base ou de sel d'addition d'acide pharmaceutiquement acceptable.

Le composé de l'invention, possédant à la fois une activité antianoxique et une activité psychotrope, peut être utilisé en thérapeutique pour le traitement des troubles de la vigilance, en particulier pour lutter contre les troubles du comportement imputables à des dommages vasculaires cérébraux et à la sclérose cérébrale en gériatrie, comme sédatif, ainsi que pour le traitement des absences dues à des traumatismes crâniens, et le traitement des états dépressifs. On peut également l'utiliser de manière générale pour le traitement des affections cérébro-vasculaires ou cardiocirculatoires.

Pour leur application en thérapeutique, le composé de l'invention peut être administré soit par voie digestive sous forme de capsules, gélules, comprimés, dragées, cachets, solution ou suspension, soit par voie parentérale sous forme de soluté stérile tamponné préparé à l'avance ou extemporanément, dans lesquels la substance active, à l'état de base ou salifiée, se trouve présent à raison de 1 mg à 300 mg de substance active par dose unitaire. La posologie quotidienne peut varier entre 1 mg et 600 mg de substance active selon l'affection.

En vue de leur application thérapeutique, le composé de l'invention est présenté sous forme de composition pharmaceutique contenant comme substance active le composé selon l'invention, éventuellement en mélange avec d'autres substances actives et les adjuvants, diluants, véhicules ou excipients habituels en pharmacie ainsi que colorants, édulcorants, agents de conservation, anti-oxydants, etc.

La préparation pharmacologique ou galénique s'effectue par les méthodes classiques, essentiellement par incorporation ou mélange des substances actives dans les adjuvants utilisés.

Dans le cas où le composé est administré sous forme de sel d'addition, il convient d'utiliser des acides pharmaceutiquement admissibles qui sont bien connus en pharmacie par exemple l'acide sulfurique ou l'acide chlorhydrique.

**Revendications pour les Etats contractants: BE, CH, DE, FR, GB, IT, LI, LU, NL, SE**

1. Le composé de formule

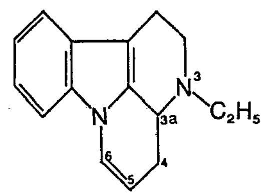

sous forme de base ou de sel d'addition d'acide organique ou minéral et sous forme d'isomère optique ou de mélange racémique.

2. L'éthyl-3-tétrahydro-1,2,3,3a-4H-indolo (3,2,1-de) (1,5)naphtyridine sous la forme de chlorhydrate.

3. Médicament caractérisé en ce qu'il comprend comme principe actif le composé selon la revendication 1 sous forme de base ou de sel d'addition d'acide pharmaceutiquement acceptable.

4. Médicament suivant la revendication 3 sous la forme de comprimés, de gélules ou de solutés injectables, dosés unitairement de 1 à 300 mg de produit actif.

5. Procédé de préparation du composé suivant la revendication 1 caractérisé en ce que la N-éthyl-hexahydro-1,2,3,3a,4,5-canthinone-6 est traitée par $LiAlH_4$, et l'alcool ainsi obtenu déshydraté par $POCl_3$ dans un mélange de benzène et de triéthylamine.

**Revendication pour l'Etat contractant: AT**

Procédé de préparation du composé de formule

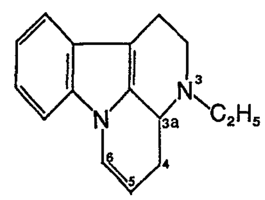

sous forme de base ou de sel d'addition d'acide organique ou minéral et sous forme d'isomère optique ou de mélange racémique, caractérisé en ce que la N-éthyl-hexahydro-1,2,3,3a,4,5-canthinone-6 de formule

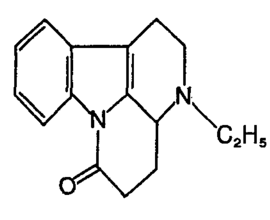

est traité par $LiAlH_4$ dans un solvent inerte, et l'alcool ainsi obtenu déshydraté par $POCl_3$ dans un mélange de benzène et de triéthylamine.

**Claims for the contracting States BE, CH, DE, FR, GB, IT, LI, LU, NL, SE.**

1. Compound of formula

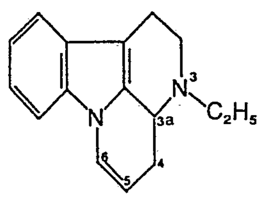

in the form of a base or of an organic or mineral acid addition salt, and in the form of the optical isomers or of the racemic mixture.

2. 3-ethyl-1,2,3,3a-tetrahydro-(3,2,1-de)-4H-indolo (1,5) naphthyridine in the form of the chlorhydrate.

3. Medicament characterized in that it contains as active substance the compound of claim 1 in the form of a base or of an acid addition salt with a pharmaceutically acceptable acid.

4. Medicament according claim 3, in the form of tablets, capsules or injectable solutes, in unitary doses of from 1 to 300 mg active substance.

5. Method for the preparation of the compound according claim 1, characterized in that the N-ethyl-1,2,3,3a,4,5-hexahydro-canthine-6-one is treated with $LiAlH_4$, and the such obtained alcohol is dehydrated by $POCl_3$ in a benzene-triethylamine mixture.

**Claim for the contracting state AT.**

Method for the preparation of the compound of formula

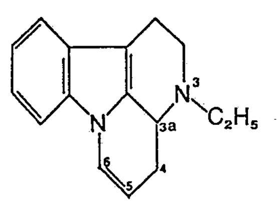

in the form of a base or of an organic or mineral acid addition salt, and in the form of the optical isomers or of the racemic mixture, characterized in that the N-ethyl-1,2,3,3a,4,5-hexahydro-canthine-6-one of formula

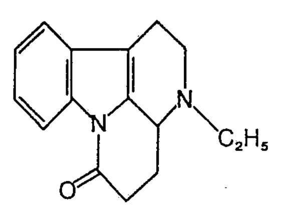

is treated with $LiAlH_4$ in an inert solvant, and the such obtained alcohol is dehydrated by $POCl_3$ in a benzene-triethylamine mixture.

**Patentansprüche für die Vertragsstaaten: BE, CH, DE, FR, GB, IT, LI, LU, NL, SE**

1. Die Verbindung der Formel

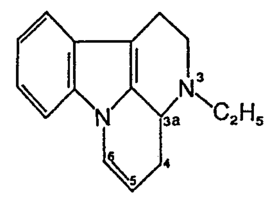

in Form der Base oder des Additionssalzes mit einer organischen oder anorganischen Säure und in Form des optischen Isomeren oder der racemischen Mischung.

2. Das 3-Äthyl-1,2,3,3a-tetrahydro-(3,2,1-de)-4H-indolo-(1,5)-naphthyridin als Chlorhydrat.

3. Arzneimittel, dadurch gekennzeichnet, dass es als Wirkkomponente die Verbindung nach Anspruch 1 in Form der Base oder des Additionssalzes mit einer pharmazeutisch annehmbaren Säure enthält.

4. Arzneimittel nach Anspruch 3 in Form von Pillen, Gelkapseln oder injizierbaren Lösungen, die einheitlich mit 1 bis 300 mg Wirkstoff dosiert sind.

5. Verfahren zur Herstellung der Verbindung nach Anspruch 1, dadurch gekennzeichnet, dass das N-Äthyl-1,2,3,3a,4,5-hexahydro-canthinon-6 mit $LiAlH_4$ behandelt und der so erhaltene Alkohol in einem Gemisch aus Benzol und Triäthylamin durch $POCl_3$ dehydratisiert wird.

**Patentanspruch für den Vertragsstaat: AT**

Verfahren zur Herstellung der Verbindung der Formel

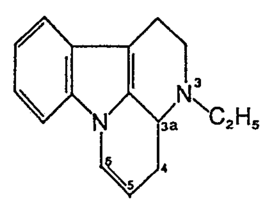

in Form der Base oder des Additionssalzes mit einer organischen oder anorganischen Säure und in Form des optischen Isomeren oder der racemischen Mischung, dadurch gekennzeichnet, dass das N-Äthyl-1,2,3,3a,4,5-hexahydro-canthinon-6 der Formel

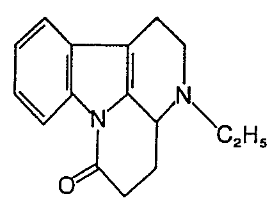

in einem inerten Lösungsmittel mit $LiAlH_4$ behandelt und der so erhaltene Alkohol in einem Gemisch aus Benzol und Triäthylamin durch $POCl_3$ dehydratisiert wird.